# EUROPEAN PATENT APPLICATION

(11) **EP 4 555 925 A1**
(43) Date of publication of application: **21.05.2025**
(21) Application number: 24213195.1
(22) Date of filing: 15.11.2024
(51) Int. Cl.: A61B 5/021, A61B 5/022

(54) **UPPER ARM BLOOD PRESSURE MONITOR**

(30) Priority: 16.11.2023 TW 112144353
(71) Applicant: Microlife Corporation, Taipei Nei-Hu 11492 (TW)
(72) Inventor: WANG, Yi-Shun, 11492 Taipei (TW); YU, Chin-Chung, 11492 Taipei (TW)
(74) Representative: Hoyng Rokh Monegier B.V.

(57) **Abstract**

The disclosure provides an upper arm blood pressure monitor (1) which comprises: a measuring host (10), a female connector (7), and a male connector (8). The measuring host (10) includes a housing (101), and the female connector (7) is disposed within the housing (101). The female connector (7) includes a gas manifold member (11), a hermetic ring (12) disposed within the gas manifold member (11), and an embedded buckle (13) also disposed within the gas manifold member (11). There is a deformable clearance between the embedded buckle (13) and the hermetic ring (12). The embedded buckle (13) includes a brim portion (131) being an annular body and a crown portion (132) extending from a periphery of the brim portion (131), wherein the crown portion (131) includes a plurality of notches (133) and the notches (133) evenly divide the crown portion (132) to correspondingly form a plurality of buckling pieces (134), and wherein a protrusion (135) is formed on a foremost end of each of the buckling pieces (134). When the female connector (7) and the male connector (8) are tightly fit to and connected with each other, the protrusions (135) of the embedded buckle (13) correspondingly engage with an annular recess (80) of the male connector (8).

## Description

### BACKGROUND OF THE INVENTION

### 1. Field of the Invention

The present disclosure is related to the fields of blood pressure monitors, in particular to an upper arm blood pressure monitor or arm-based sphygmomanometer having a male connector and a female connector.

### 2. Description of Related Art

The upper arm blood pressure monitor or arm-based sphygmomanometer is a widely used device in the medical field. Its operation primarily relies on an air pump within the sphygmomanometer that inflates and deflates the cuff attached to the patient's arm by delivering and releasing air. A pressure sensor then measures the pressure in the cuff and provides the sphygmomanometer with the necessary data. Since the patient's life and health may be directly or indirectly impacted by blood pressure measurements, any issue with the airtight stability of the male connectors can lead to inaccurate readings. Additionally, there is a risk of the cuff being mistakenly connected to other medical devices, or the sphygmomanometer may not be properly attached to other equipment. Such errors could cause medical personnel to implement incorrect treatments or procedures. To address these concerns, adhering to the international standard ISO 80369-5, 'Small-bore connectors for liquids and gases in healthcare applications,' can effectively reduce these risks and improve safety.

Furthermore, the plug-in connector of the current upper arm blood pressure monitor is just used to extend the connection between the tube (or conduit) and cuff further to the host of the sphygmomanometer. So, its function is quite simple. Furthermore, since there are more restrictions on the required specification of a male connector, most of the design changes focus on the structural design of the female connector. In addition, the female connector is installed inside the main host and is extended and connected to each of an air pump and tubes. Therefore, various manufacturers inevitably have different considerations in structural design of the female connector. Regardless of how to avoid incorrectly plugging the connector into other devices, it is still a fundamental requirement to maintain better connection stability after the male connector connects with the female connector. Moreover, in different applicable fields, the same mechanism design basis can still be adapted to the implementation through slight changes in the mechanism design even under different pull-out force requirements. Such modification also has a beneficial effect on the overall design cost consideration in practice.

In view of above, the applicant conceived and proposed an upper arm blood pressure monitor with a newly changed female connector to comply with international standards, maintain a better and stable plug-in effect, reduce the risk of air leakage, and obtain better results of blood pressure measurement.

### SUMMARY OF THE INVENTION

The aspect of the present disclosure is to improve the structure of the female connector of the upper arm blood pressure monitor or arm-based sphygmomanometer, and to use the housing of the upper arm blood pressure monitor to retain the female connector. Since the housing has a large volume and has better stability after being stressed, even if the plug-in action is repeated, it will not cause the female connector to shake or detach. In addition, for the internal design of the female connector, the present disclosure also proposes that the male connector can be stably engaged with the female connector. Moreover, in response to the needs of different applicable fields, the structural design can be slightly adjusted to satisfy the different pull-out forces using an embedded buckle matched with a hermetic ring (such as O-ring) so that such disclosure has better air sealing effect.

In one aspect of the present disclosure, an upper arm blood pressure monitor comprises: a measuring host including a housing with a first insertion opening, and a latching slot formed within the housing corresponding to the first insertion opening; a female connecter disposed inside the housing, and the female connector comprising: a gas manifold member including a latching section, a front end pipe body, a rear end pipe body and a branch pipe that are communicated with each other in sequence along a axial direction and are integrally formed, the gas manifold member including a second insertion opening, wherein the latching section is inserted and fixed in the latching slot, and the second insertion opening corresponds to the first insertion opening; a hermetic ring disposed in the gas manifold member; and an embedded buckle disposed in the gas manifold member and between the second insertion opening and the hermetic ring, the embedded buckle comprising: a brim portion being an annular body and including a third insertion opening, wherein the brim portion abuts an inner wall of the latching section, and the brim portion blocked and limited by a portion of the housing around the first insertion opening; and a crown portion extending from a periphery of the third insertion opening of the brim portion, wherein the crown portion has a plurality of notches, the notches evenly divide the crown portion to form a plurality of buckling pieces, and each of the buckling pieces has an inner surface and an outer surface; wherein the inner surfaces of the buckle pieces are respectively provided with protrusions, and each protrusion has a height; and a male connector including an annular recess, and connected to an cuff with a tube; wherein when the protrusions of the embedded buckle are correspondingly clamped into the annular recess of the male connector, the male connector and the female connector are tightly matched with each other.

In yet another aspect of the present disclosure, the inner wall of the latching section has a first inner diameter and a second inner diameter, the first inner diameter is larger than the second inner diameter; an inner wall of the front end pipe body has a third inner diameter, a fourth inner diameter and a fifth inner diameter, the third inner diameter is greater than the fourth inner diameter, the fourth inner diameter is greater than the fifth inner diameter, and the third inner diameter is substantially equal to the second inner diameter so that a junction where the latching section and the front end pipe body connect with each other is coplanar; and an inner wall of the rear end pipe body has a sixth inner diameter, and the sixth inner diameter is substantially equal to the fifth inner diameter so that a junction where the front end pipe body and the rear end pipe body connect with each other is coplanar.

In another aspect of the present disclosure, the hermetic ring is disposed at a location of the inner wall of the front end pipe body that is between the third inner diameter and the fourth inner diameter.

In another aspect of the present disclosure, an offset clearance is from the outer surfaces of the buckling pieces to the second inner diameter, and a width of the offset clearance is greater than the height of the protrusions.

In another aspect of the present disclosure, the gas manifold member and the embedded buckle have an elastic modulus greater than 700 MPa, and the hermetic ring has an elastic modulus less than 700 MPa.

In yet another aspect of the present disclosure, the protrusions are wedge-shaped.

In yet another aspect of the present disclosure, the notches have a length ranging from 1.9 mm to 3.5 mm.

In another aspect of the present disclosure, the brim portion abuts and is welded to a location of the inner wall of the latching section that is between the first inner diameter and the second inner diameter.

In another aspect of the present disclosure, ultrasonic welding is used to weld the brim portion and the latching section together.

In another aspect of the present disclosure, the embedded buckle is made of thermoplastic polyester elastomer.

In another aspect of the present disclosure, an outer contour of the front end pipe body is circular, and the latching slot has an arc neck which the front end pipe body abuts.

In another aspect of the present disclosure, two protruding ribs is disposed on an outer surface of the latching section, and the housing extends inwards and is partially clamped between the two protruding ribs so that the housing covers one of the protruding ribs.

In another aspect of the present disclosure, a female connecter for an upper arm blood pressure monitor, wherein a first insertion opening is exposedly disposed on a housing of the upper arm blood pressure monitor and the female connecter is disposed within the housing, the female connecter comprises: a gas manifold member including a latching section, a front end pipe body, a rear end pipe body and a branch pipe that are communicated with each other in sequence along a axial direction and are integrally formed, the gas manifold member including a second insertion opening, the second insertion opening disposed in correspondence with the first insertion opening; a hermetic ring disposed in the gas manifold member; and an embedded buckle disposed in the gas manifold member and between the second insertion opening and the hermetic ring, the embedded buckle comprising: a brim portion being an annular body and including a third insertion opening, wherein the brim portion abuts an inner wall of the latching section, and the brim portion blocked and limited by a portion of the housing around the first insertion opening; and a crown portion extending from a periphery of the third insertion opening of the brim portion, wherein the crown portion has a plurality of notches, the notches evenly divide the crown portion to form a plurality of buckling pieces, and each of the buckling pieces has an inner surface and an outer surface; wherein the inner surfaces of the buckle pieces are respectively provided with protrusions, and each protrusion has a height.

In view of above, the upper arm blood pressure monitor of the present disclosure uses the housing to press against the female connector. Accordingly, since the housing has a large volume, the structural stability of the connection between the female connector and male connector is well increased during the plugging and unplugging operations so that the male connector is difficult to shake and loose when connected to the gas manifold member. Regarding the internal design of the female connector, the present disclosure also proposes an embedded buckle that can stably engage with the male connector and meet different pull-out force requirements just by slightly adjusting the structural design in response to the needs of different fields. Based on the stability when the gas manifold member is assembled with the housing, embedded buckle, and hermetic ring and connected with the male connector in fluidically communication, the gas manifold member is designed to have a latching section, front-end pipe body and rear-end pipe body in sequence. Furthermore, through the preferable inner diameters, the gas manifold member has excellent structural stability after being assembled with the foregoing components, eliminating each component from shaking and shifting during the plugging and unplugging action of the male connector or blood pressure measurement. Therefore, the sphygmomanometer has excellent plugging stability, gas tightness and flow stability. In addition, in order to avoid the dangerous situation of mis-plugging, the present disclosure also proposes to limit the elastic modulus of each component so that the hermetic ring located at the rearmost position in the structure of the female connector can be used as a deformable component under pressed force. The deformation can be deemed as the basis for judging whether the plug is correct or not, thus eliminating the misjudgment caused by the object being deformed due to force when the male connector is inserted into the front end of the female connector. Furthermore, in order to improve the strength and stability of the connection during plugging, it can be achieved by making the protrusions into wedge shapes. In addition, in order to meet the more common pulling force requirements of the sphygmomanometer, it can be achieved by adjusting the length, number and so on of the notches. Moreover, considering the assembly strength of the gas manifold member and the embedded buckle, in order to reduce the occurrence of component detachment caused by repeated plugging and unplugging, the brim portion of the embedded buckle and the inner wall of the gas manifold member are welded to each other through, for example, ultrasonic waves. The embedded buckle is made of thermoplastic polyester elastomer to ensure its rigidity and prevent it from being deformed during plugging and causing the misjudgment of correct plugging. Also, there is a slight elastic margin or widow so that the male connector can be smoothly plugged and unplugged. In addition, based on the assembly stability of the gas manifold member and the inside of the housing, the present disclosure also proposes that the housing can be partially covered by the protruding ribs on the outer surface of the latching section, and the front end pipe body and the latching slots are further designed into a circular outline to improve the close contact between them.

### BRIEF DESCRIPTION OF THE DRAWINGS

In order to sufficiently understand the essence, advantages and the preferred embodiments of the present invention, the following detailed description will be more clearly understood by referring to the accompanying drawings.
FIG. 1 is a schematic structural diagram of an upper arm blood pressure monitor and a cuff according to an embodiment of the present disclosure.
FIG. 2 is a schematic structural diagram of an upper arm blood pressure monitor according to an embodiment of the present disclosure.
FIG. 3A is a partial cross-sectional view of the upper arm blood pressure monitor according to the embodiment of the present disclosure along the line A-A in FIG. 2.
FIG. 3B is an enlarged structural view of the area marked C in FIG. 2 of the upper arm blood pressure monitor according to the embodiment of the present disclosure.
FIG. 4 is an exploded schematic diagram of the three-dimensional structure of the partial housing, the gas manifold member, the hermetic ring and the embedded buckle according to the embodiment of the present disclosure.
FIG. 5 is an exploded schematic diagram of the three-dimensional structure of the gas manifold member, the hermetic ring and the embedded buckle according to the embodiment of the present disclosure.
FIG. 6 is a schematic three-dimensional structural diagram of an embedded buckle according to an embodiment of the present invention.
FIG. 7 is a cross-sectional view of the embedded buckle according to the embodiment of the present invention along the line B-B in FIG. 6.
FIG. 8 is a schematic assembly diagram of the three-dimensional structure of the partial housing, the gas manifold member, the hermetic ring and the embedded buckle according to an embodiment of the present disclosure.

### DETAILED DESCRIPTION OF THE INVENTION

The following description shows the preferred embodiments of the present disclosure. The present invention is described below by referring to the embodiments and the figures. Thus, the present disclosure is not intended to be limited to the embodiments shown but is to be accorded the principles disclosed herein. Furthermore, that various modifications or changes in light thereof will be suggested to persons skilled in the art and are to be included within the spirit and purview of this application and scope of the appended claims.

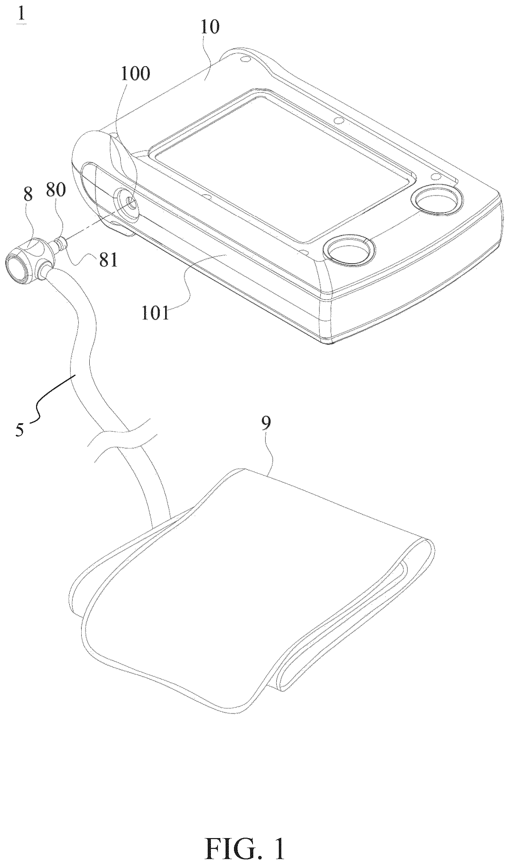

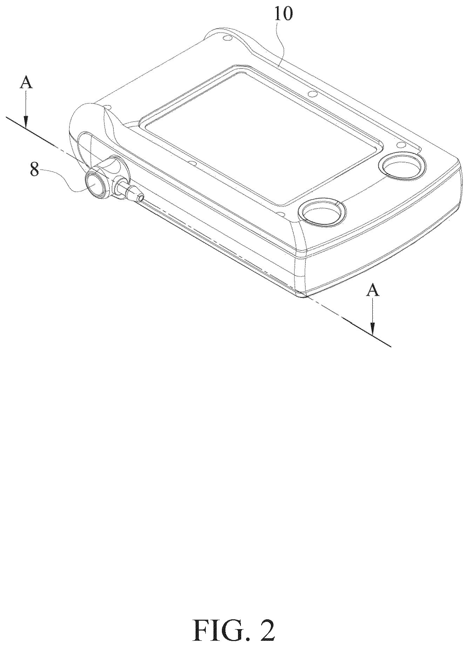

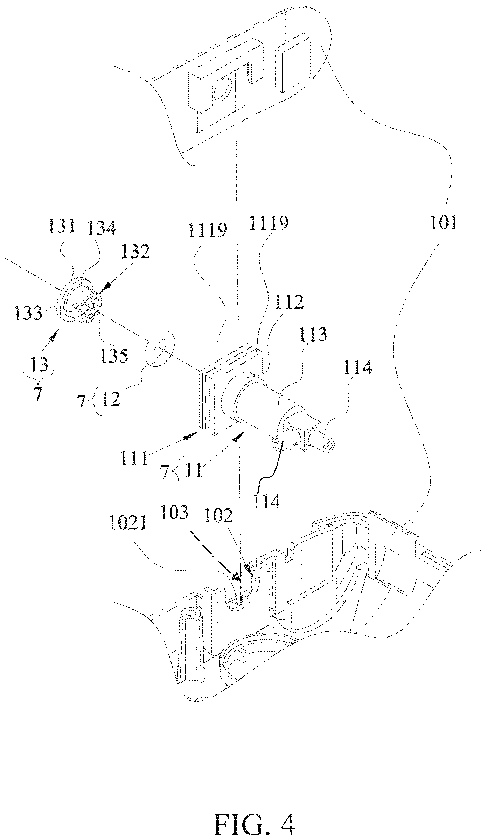

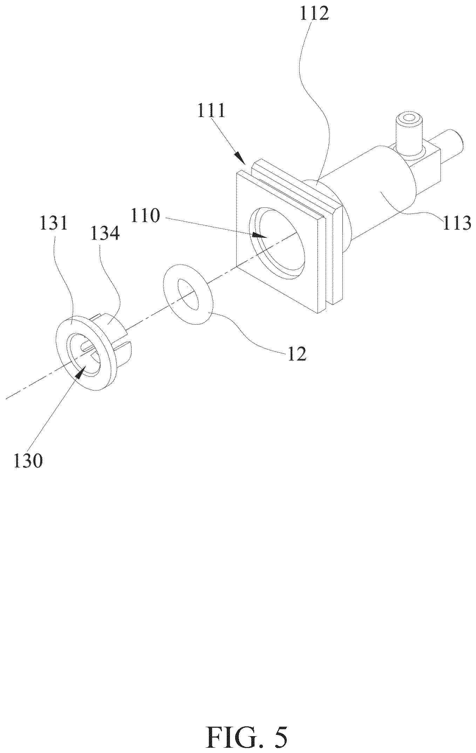

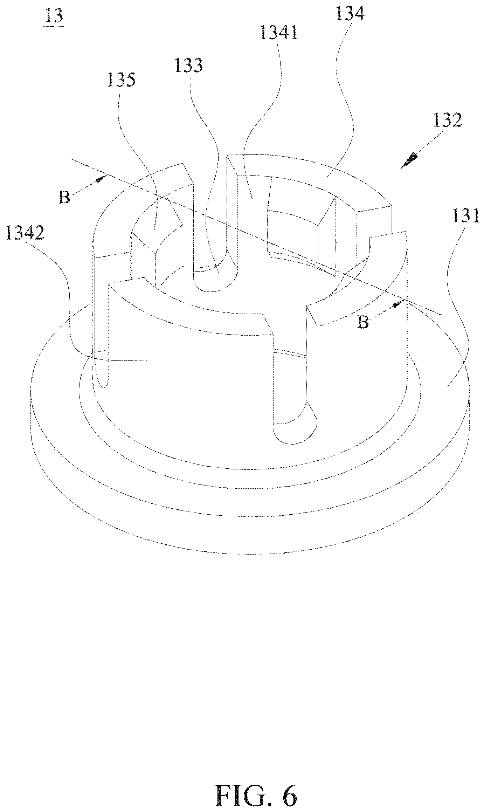

Referring to figures 1 to 6, they respectively are respectively a schematic structural diagram of an upper arm blood pressure monitor and a cuff, a schematic structural diagram of an upper arm blood pressure monitor, a partial cross-sectional view of the upper arm blood pressure monitor along the line A-A in FIG. 2, an enlarged structural view of the area marked C in FIG. 2 of the upper arm blood pressure monitor, an exploded schematic diagram of the three-dimensional structure of the partial housing, the gas manifold member, the hermetic ring and the embedded buckle, a schematic three-dimensional structural diagram of an embedded buckle, a cross-sectional view of the embedded buckle along the line B-B in FIG. 6, and a schematic assembly diagram of the three-dimensional structure of the partial housing, the gas manifold member, the hermetic ring and the embedded buckle. As shown in these figures, the upper arm blood pressure monitor 1 proposed by the present disclosure has a measuring host 10, a female connector 7, and a male connector 8. The upper arm blood pressure monitor 1 is used for wrapping a cuff 9 at the upper arm of the user, which is fluidically connected with a tube 5 (or conduit). A person having ordinary knowledge in the art of the present disclosure will know that the cuff 9 further has a single airbag (bladder) inside. The cuff 9 is tightly connected to the female connector 7 through the male connector 8, and the male connector 8 has an annular recess 80 and a hermetic member 81. Particularly, the female connector 7 is composed of a gas manifold member 11, a hermetic ring 12 and an embedded buckle 13 as described later. When the user uses the upper arm blood pressure monitor 1, the upper arm blood pressure monitor 1 can be activated to inflate/deflate gas into the cuff 9 to complete blood pressure measurement or other assessments such as arrhythmia or atrial fibrillation. In this embodiment, the measurement of the upper arm blood pressure monitor 1 can be an inflated measurement or a deflated measurement. Furthermore, in this embodiment, the measuring host 10 of the upper arm blood pressure monitor 1 has a housing 101, and a first insertion opening 100 is exposedly disposed on the side of the housing 101 and is used to allow the male connector 8 to plug therethrough. The female connector 7 is disposed in the housing 101, and a latching slot 102 is formed within the housing 101 corresponding to the first insertion opening 100. Furthermore, the gas manifold member 11 is communicated with and disposed in the housing 101 corresponding to the first insertion opening 100. Since the male connector 8 is connected to the female connector 7 within the measuring host 10 through the first insertion opening 100 to perform blood pressure measurement. The purpose of the latching slot 102 is to hold the gas manifold member 11 so that the gas manifold member 11 is firmly disposed in the measuring host 10 and the male connector 8 is prevented from shaking after being connected thereto.

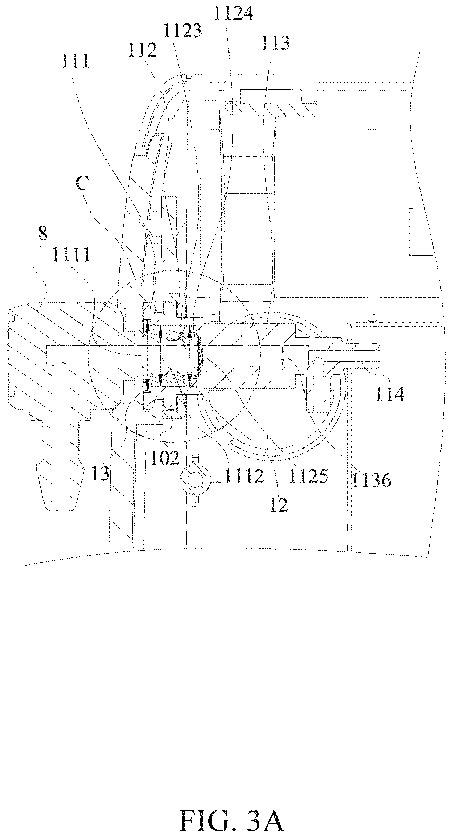

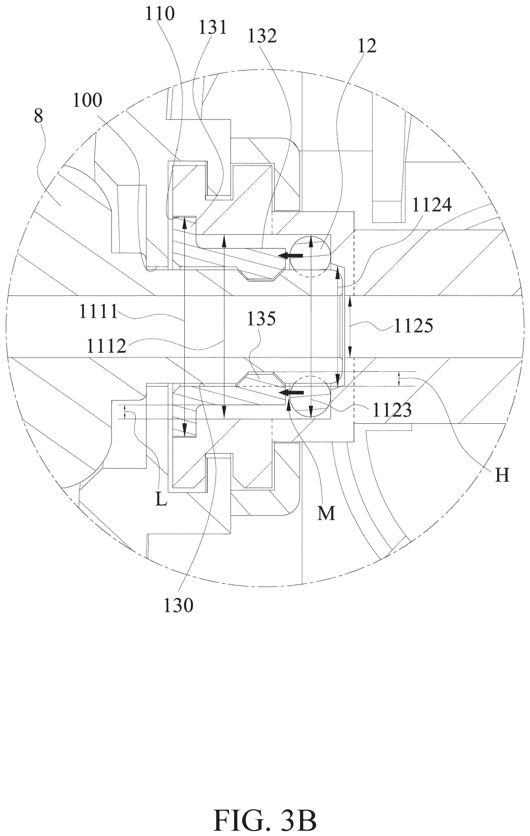

Please refer to FIGS. 3A and 3B again. As mentioned above, the gas manifold member 11 has a latching section 111, a front end pipe body 112, a rear end pipe body 113, and a branch pipe 114 that are communicated with each other in sequence along an axial direction and are integrally formed in one piece. The gas manifold member 11 has a second insertion opening 110. The second insertion opening 110 is concentrically arranged corresponding to the location of the first insertion opening 100. The latching section 111 is adjacent to the insertion opening 103 and firmly inserted in the latching slot 102. The branch pipe 114 is provided for connection with the air pump in the measuring host 10. The inner wall of the latching section 111 has a first inner diameter 1111 and a second inner diameter 1112, and the first inner diameter 1111 is larger than the second inner diameter 1112. The inner wall of the front end pipe body 112 has a third inner diameter 1123, a fourth the inner diameter 1124, and a fifth inner diameter 1125, the third inner diameter 1123 is greater than the fourth inner diameter 1124, the fourth inner diameter 1124 is greater than the fifth inner diameter 1125, and the third inner diameter 1123 is equal to the second inner diameter 1112 so that the junction where the latching section 111 and the front end pipe body 112 connect with each other is coplanar. The inner wall of the rear end pipe body 113 has a sixth inner diameter 1136, and the sixth inner diameter 1136 is equal to the fifth inner diameter 1125 so that the junction where the front end pipe body 112 and the rear end pipe body 113 connect with each other is coplanar. Furthermore, the hermetic ring 12 is disposed at the location of the inner wall of the front end pipe body 122 that is between the third inner diameter and the fourth inner diameter. The embedded buckle 13 is disposed within the gas manifold member 11 and is located between the second insertion opening 110 and the hermetic ring 12. There is a deformable clearance between the hermetic ring 12 and the embedded buckle 13 as marked with M in FIG. 3B. In this regard, the hermetic ring 12 is shown in a load-free non-deformation state in FIGS. 3A and 3B. During the state, it can be seen that there is a deformable clearance between the hermetic ring 12 and the embedded buckle 13. When the male connector 8 reaches the hermetic ring 12 during plugging, the hermetic ring 12 will deform in the direction of the arrow shown in FIG. 3B and fill the deformable clearance (not shown in the figure). Generally speaking, the internal structures of the latching section 111, the front end pipe body 112, and the rear end pipe body 113 are designed to match the configurations of the hermetic ring 12 and the embedded buckle 13. Through this snap setting manner, the hermetic ring 12 and the embedded buckle 13 can be quickly positioned, and the hermetic ring 12 and the embedded buckle 13 can be prevented from being unexpectedly shifted in position by exerted force during the insertion and removal actions while maintaining better air tightness. In the embodiment, the gas manifold member 11 is formed in one piece, which can prevent gas leakage from affecting the pressure detection results. When the hermetic member 81 of the male connector 8 is inserted into the hermetic ring 12, the deformable clearance can be used as a deformable margin for the hermetic ring 12 after being squeezed to improve the overall airtight effect.

Further, the embedded buckle 13 includes a brim portion 131 and a crown portion 132. The brim portion 131 is an annular body and has a third insertion opening 130. The brim portion 131 is disposed at the location of the inner wall of the latching section 111 that is between the first inner diameter 1111 and the second inner diameter 1112. The crown portion 132 extends from the periphery of the third insertion opening 130 of the brim portion 131. In this embodiment, the crown portion 132 has four notches 133, and the notches 133 evenly divide the crown portion 132 to form four buckling pieces 134. Therefore, in the consideration of the pulling force requirements under various application fields, for example, if the pulling force standards for household or sphygmomanometers are different, the pulling force can be adjusted through different numbers of notches 133.

Further, the buckling pieces 134 respectively have an inner surface 1341 and an outer surface 1342, wherein the inner surface 1341 of each buckling piece 134 is provided with a protrusion 135. There is an offset clearance from the outer surfaces 1342 of the buckling pieces 134 to the second inner diameter 1112 as marked with L in FIG. 3B, and the width of the offset clearance is greater than the height of the protrusions 135, and the height of the protrusions 135 is marked with H in FIG. 3B. Particularly, the diameter of the first insertion opening 100 is smaller than that of the second insertion opening 110 so that the brim portion 131 of the embedded buckle 13 is blocked and limited by a part of the housing 101 around the first insertion opening 100. When the male connector 8 is completely inserted into the female connector 7, each protrusion 135 will be correspondingly stuck in the annular recess 80 of the male connector 8. In this way, the airtightness and assembly stability of the male connector 8 can be greatly improved after it is inserted into the female connector 7 within the upper arm blood pressure monitor 1. In addition, the elastic modulus of the gas manifold member 11 and the embedded buckle 13 is greater than 700 MPa, and the elastic modulus of the hermetic ring 12 is less than 700 MPa. Since the hermetic ring 12 within the female connector 7 of the upper arm blood pressure monitor 1 can serve as the deformable component after being compressed, it is possible to achieve that the hermetic ring 12 within the female connector 7 is deformed by the male connector 8 just during plugging. The deformation can be deemed as the basis for judging whether the connection between the male connector 8 and the female connector 7 is adequate or not (in compliance with ISO 80369-5 specifications).

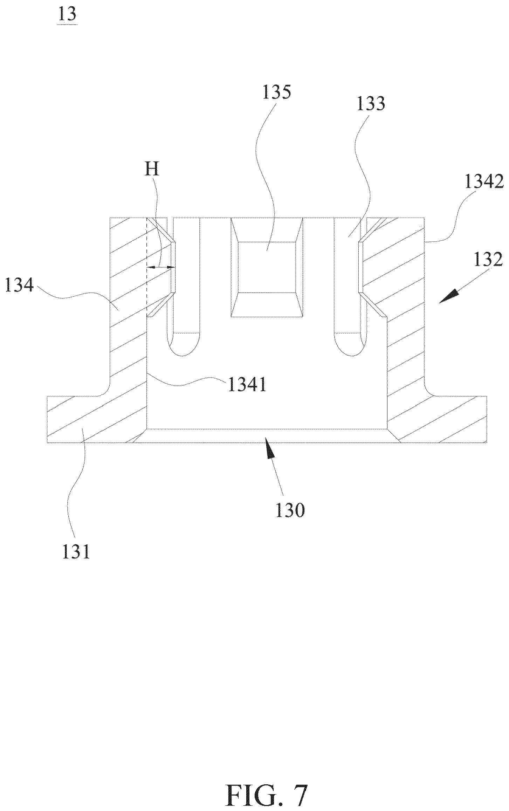

Referring to FIG. 7, it is a cross-sectional view of the embedded buckle according to the embodiment of the present disclosure along the line B-B in FIG. 6. In order to improve the airtightness after the male connector 8 is plugged into the female connector 7, the shape of the protrusions 135 can optionally be wedge, but the disclosure is not limited to this. After the male connector 8 is inserted into the gas manifold member 11 through the second insertion opening 110, the annular recess 80 and the protrusions 135 of the embedded buckle 13 are tightly engaged and connected with each other. The assembly between the male connector 8 and the female connector 7 is less likely to loosen due to insufficient anti-unplugging force of the male connector 8 during the inflation and deflation of the cuff.

The pulling force requirement of the upper arm blood pressure monitor 1 can be varied by adjusting the length of the notches 133 in addition to adjusting the number of the notches 133 as described above. For example, using the length of the notches 133 as the controlling factor, when the length of the notches 133 is longer, it can be applied to sphygmomanometers with smaller pulling force requirements, such as consumer or household ones. By contrast, if the length of the notches 133 is short, it can be suitable for sphygmomanometers with larger pulling force requirements, such as professional or medical ones. In this embodiment, the length of the notches 133 ranges from 1.9 mm to 3.5 mm so as to meet the pulling force requirements of the above common sphygmomanometers. For example, when the length of the grooves 133 is 1.9 mm, the embedded buckle 13 can achieve an interlocking force of about 35 Newtons, which can meet the requirements of a medical sphygmomanometer. When the length of the notches 133 is 3.5 mm, the embedded buckle 13 can reach an interlocking force of about 3 Newtons, which is suitable for household sphygmomanometers. Of course, the length of the notches 133 can be in the range of 1.9 mm to 3.5 mm, and can be changed according to actual pulling force requirements. It is further emphasized that this embodiment only illustrates one of the preferable structure of the embedded buckle 13. That is, the number of the notches 133 is four, and the length of them is 3 mm. Based on this structure, another factor that can change the interlocking force of the embedded buckle 13 is introduced, that is, the number of the notches 133, so that the number and length of the notches 133 meet the above conditions, Accordingly, the embedded buckle 13 can achieve a interlocking force of about 10 Newtons. This or the specific examples of the length or number of the notches 133 can also prove that the embedded buckle 13 can indeed adapt to different fields and meet different required pulling forces by slightly adjusting the structural design of the notches 133.

In order to improve the assembly strength and structural stability of the embedded buckle 13 and the gas manifold member 11 during use, preferably, the brim portion 131 can abut upon the location of the inner wall of the latch section 111 between the first inner diameter 1111 and the second inner diameter 1112 and is further firmly welded together, for example, through ultrasonic welding. In this way, it is possible to avoid the loosening of components caused by the plugging and unplugging actions of the male connector 8.

The embedded buckle 13 needs to be slightly elastic without breaking due to stress and has an elastic modulus greater than 700Mpa. Preferably, the embedded buckle 13 can be made of thermoplastic polyester elastomer material so as to make the embedded buckle 13 have appropriate structural performance. In this embodiment, the elastic modulus of thermoplastic polyester elastomer (THERMOPLASTIC POLYESTER ESTER ELASTOMER, TPEE) is about 770 MPa.

In order to improve the assembly strength and stability of the gas manifold member 11 and the latching slot 102 of the housing 101, the outer contour of the front end pipe body 112 can be circular, and the latching slot 102 has an arc neck 1021 for the front end pipe body 112 to abut. Through the circular outline and the design of the arc neck 1021, when the front end pipe body 112 abuts the latching slot 102, the mutually abutting parts form a closer contact. In addition to the function of firmly supporting the front end pipe body 112 from below, it can also have a reliable retaining effect on the side according to the circular and arc-shaped contours. In this regard, the installed gas manifold member 11 is enhanced to maintain the stability of the connection of the male connector 8 and female connector 7 during repeatedly plugging and unplugging.

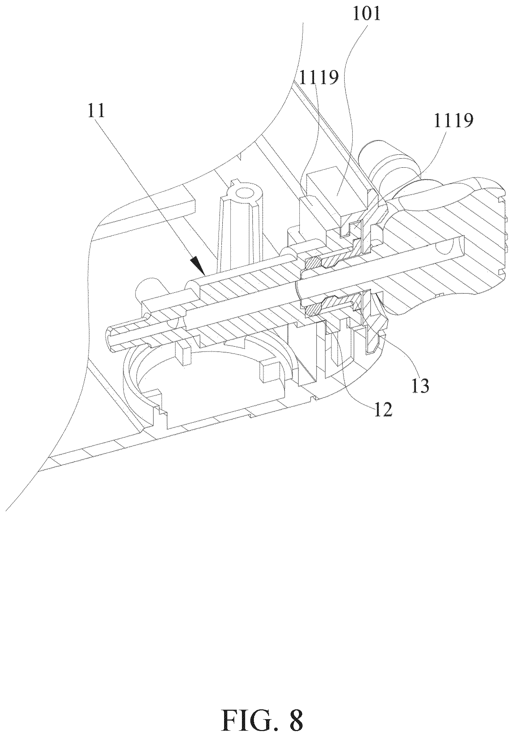

Further referring to FIG. 8, this figure is a schematic assembly diagram of the three-dimensional structure of the partial structure in the housing, the gas manifold member, the hermetic ring and the embedded buckle according to the embodiment of the present disclosure. Preferably, the outer surface of the latching section 111 is provided with two protruding ribs 1119, and a portion of the housing 101 extends inwardly and is partially clamped between the two protruding ribs 1119 so that the portion of the housing 101 is covered by one of the protruding ribs 1119. In this way, the assembly of the housing 101 and the gas manifold member 11 can be made more stable since the portion of the housing 101 extends inwardly and is clamped by the two protruding ribs 1119. Furthermore, the portion of the housing 101 is further covered by one of the protruding ribs 1119, and such a configuration can enhance the assembly stability of the housing 101 and the gas manifold member 11 to be in a strong assembly state, thereby greatly reducing the probability of components falling off or slipping during use.

In view of above, the upper arm blood pressure monitor of the present disclosure uses the housing to press against the female connector. Accordingly, since the housing has a large volume, the structural stability of the connection between the female connector and male connector is well increased during the plugging and unplugging operations so that the male connector is difficult to shake and loose using few buckle pieces against push and pull-out forces. Regarding the internal design of the female connector, the present disclosure also proposes an embedded buckle that can stably engage with the male connector and meet different pull-out force requirements just by slightly adjusting the structural design in response to the needs of different fields. Based on the stability when the gas manifold member is assembled with the housing, embedded buckle, and hermetic ring and connected with the male connector in fluidically communication, the gas manifold member is designed to have a latching section, front-end pipe body and rear-end pipe body in sequence. Furthermore, through the preferable inner diameters, the gas manifold member has excellent structural stability after being assembled with the foregoing components, eliminating each component from shaking and shifting during the plugging and unplugging actions of the male connector or blood pressure measurement. Therefore, the sphygmomanometer has excellent plugging stability, gas tightness and flow stability. In addition, in order to avoid the dangerous situation of mis-plugging, the present disclosure also proposes to limit the elastic modulus of each component so that the hermetic ring located at the rearmost position in the structure of the female connector can be used as a deformable component under pressed force. The deformation can be deemed as the basis for judging whether the plug is correct or not, thus eliminating the misjudgment caused by the object being deformed due to force when the male connector is inserted into the front end of the female connector. Furthermore, in order to improve the strength and stability of the connection during plugging, it can be achieved by making the protrusions into wedge shapes. In addition, in order to meet the more common pulling force requirements of the sphygmomanometer, it can be achieved by adjusting the length, number and so on of the notches. For example, the length of the notches 133 can be in the range of 1.9 mm to 3.5 mm. Moreover, in another modification to the embedded buckle, the number of the notches 133 is four, and the length of them is 3 mm. Moreover, considering the assembly strength of the gas manifold member and the embedded buckle, in order to reduce the occurrence of component detachment caused by repeated plugging and unplugging, the brim portion of the embedded buckle and the inner wall of the gas manifold member are welded to each other through, for example, ultrasonic waves. The embedded buckle is made of thermoplastic polyester elastomer to ensure its rigidity and prevent it from being deformed during plugging and causing the misjudgment of correct plugging. Also, there is a slight elastic margin or widow so that the male connector can be smoothly plugged and unplugged. In addition, based on the assembly stability of the gas manifold member and the inside of the housing, the present disclosure also proposes that the housing can be partially covered by the protruding ribs on the outer surface of the latching section, and the front end pipe body and the latching slots are further designed into a circular outline to improve the close contact between them.

Although the present invention is written with respect to specific embodiments and implementations, various changes and modifications may be suggested to a person having ordinary skill in the art. It is intended that the present disclosure encompass such changes and modifications that fall within the scope of the appended claims.

## Claims

1. An upper arm blood pressure monitor (1) comprising:
a measuring host (10) including a housing (101) with a first insertion opening (100), and a latching slot (102) formed within the housing (101) corresponding to the first insertion opening (100);
a female connecter (7) disposed inside the housing (101), and the female connector (7) comprising:
a gas manifold member (11) including a latching section (111), a front end pipe body (112), a rear end pipe body (113) and a branch pipe (114) that are communicated with each other in sequence along an axial direction and are integrally formed, the gas manifold member (11) including a second insertion opening (110), wherein the latching section (111) is inserted and fixed in the latching slot (102), and the second insertion opening (110) corresponds to the first insertion opening (100);
a hermetic ring (12) disposed in the gas manifold member (11); and
an embedded buckle (13) disposed in the gas manifold member (11) and between the second insertion opening (110) and the hermetic ring (12), the embedded buckle (13) comprising:
a brim portion (131) being an annular body and including a third insertion opening (130), wherein the brim portion (131) abuts an inner wall of the latching section (111), and the brim portion (131) blocked and limited by a portion of the housing (101) around the first insertion opening (100); and
a crown portion (132) extending from a periphery of the third insertion opening (130) of the brim portion (131), wherein the crown portion (132) has a plurality of notches (133), the notches (133) evenly divide the crown portion (132) to form a plurality of buckling pieces (134), and each of the buckling pieces (134) has an inner surface (1341) and an outer surface (1342); wherein the inner surfaces (1341) of the buckle pieces (134) are respectively provided with protrusions (135), and each protrusion (135) has a height (H); and
a male connector (8) including an annular recess (80), and connected to an cuff (9) with a tube (5);
wherein when the protrusions (135) of the embedded buckle (13) are correspondingly clamped into the annular recess (80) of the male connector (8), the male connector (8) and the female connector (7) are tightly matched with each other.

2. The upper arm blood pressure monitor (1) according to Claim 1, wherein the inner wall of the latching section (111) has a first inner diameter (1111) and a second inner diameter (1112), the first inner diameter (1111) is larger than the second inner diameter (1112); an inner wall of the front end pipe body (112) has a third inner diameter (1123), a fourth inner diameter (1124) and a fifth inner diameter (1125), the third inner diameter (1123) is greater than the fourth inner diameter (1124), the fourth inner diameter (1124) is greater than the fifth inner diameter (1125), and the third inner diameter (1123) is substantially equal to the second inner diameter (1122) so that a junction where the latching section (111) and the front end pipe body (112) connect with each other is coplanar; and an inner wall of the rear end pipe body (113) has a sixth inner diameter (1136), and the sixth inner diameter (1136) is substantially equal to the fifth inner diameter (1125) so that a junction where the front end pipe body (112) and the rear end pipe body (113) connect with each other is coplanar.

3. The upper arm blood pressure monitor (1) according to Claim 2, wherein the hermetic ring (12) is disposed at a location of the inner wall of the front end pipe body (112) that is between the third inner diameter (1123) and the fourth inner diameter (1124).

4. The upper arm blood pressure monitor (1) according to Claim 2, wherein an offset clearance (L) is from the outer surfaces (1342) of the buckling pieces (134) to the second inner diameter (1112), and a distance of the offset clearance (L) is greater than the height (H) of the protrusions (135).

5. The upper arm blood pressure monitor (1) according to Claim 1, wherein the gas manifold member (11) and the embedded buckle (13) have an elastic modulus greater than 700 MPa, and the hermetic ring (12) has an elastic modulus less than 700 MPa.

6. The upper arm blood pressure monitor (1) according to Claim 1, wherein the protrusions (135) are wedge-shaped.

7. The upper arm blood pressure monitor (1) according to Claim 1, wherein the notches (133) have a length ranging from 1.9 mm to 3.5 mm.

8. The upper arm blood pressure monitor (1) according to Claim 2, wherein the brim portion (131) abuts and is welded to a location of the inner wall of the latching section (111) that is between the first inner diameter (1111) and the second inner diameter (1112).

9. The upper arm blood pressure monitor (1) according to Claim 8, wherein ultrasonic welding is used to weld the brim portion (131) to the latching section (111).

10. The upper arm blood pressure monitor (1) according to Claim 1, wherein the embedded buckle (13) is made of thermoplastic polyester elastomer.

11. The upper arm blood pressure monitor (1) according to Claim 1, wherein an outer contour of the front end pipe body (112) is circular, and the latching slot (102) has an arc neck (1021) which the front end pipe body (112) abuts.

12. The upper arm blood pressure monitor (1) according to Claim 1, wherein two protruding ribs (1119) is disposed on an outer surface of the latching section (111), and the housing (101) extends inwards and is partially clamped between the two protruding ribs (1119) so that the housing (101) covers one of the protruding ribs (1119).

13. A female connecter (7) for an upper arm blood pressure monitor (1), wherein a first insertion opening (100) is exposedly disposed on a housing (101) of the upper arm blood pressure monitor (1) and the female connecter (7) is disposed within the housing (101), the female connecter (7) comprising:
a gas manifold member (11) including a latching section (111), a front end pipe body (112), a rear end pipe body (113) and a branch pipe (114) that are communicated with each other in sequence along an axial direction and are integrally formed, the gas manifold member (11) including a second insertion opening (110), the second insertion opening (110) disposed in correspondence with the first insertion opening (100);
a hermetic ring (12) disposed in the gas manifold member (11); and
an embedded buckle (13) disposed in the gas manifold member (11) and between the second insertion opening (110) and the hermetic ring (12), the embedded buckle (13) comprising:
a brim portion (131) being an annular body and including a third insertion opening (130), wherein the brim portion (131) abuts an inner wall of the latching section (111), and the brim portion (131) blocked and limited by a portion of the housing (101) around the first insertion opening (100); and
a crown portion (132) extending from a periphery of the third insertion opening (130) of the brim portion (131), wherein the crown portion (132) has a plurality of notches (133), the notches (133) evenly divide the crown portion (132) to form a plurality of buckling pieces (134), and each of the buckling pieces (134) has an inner surface (1341) and an outer surface (1342); wherein the inner surfaces (1341) of the buckle pieces (134) are respectively provided with protrusions (135), and each protrusion (135) has a height (H).

14. The female connecter (7) according to Claim 13, wherein the inner wall of the latching section (111) has a first inner diameter (1111) and a second inner diameter (1112), the first inner diameter (1111) is larger than the second inner diameter (1112); an inner wall of the front end pipe body (112) has a third inner diameter (1123), a fourth inner diameter (1124) and a fifth inner diameter (1125), the third inner diameter (1123) is greater than the fourth inner diameter (1124), the fourth inner diameter (1124) is greater than the fifth inner diameter (1125), and the third inner diameter (1123) is substantially equal to the second inner diameter (1112) so that a junction where the latching section (111) and the front end pipe body (112) connect with each other is coplanar; and an inner wall of the rear end pipe body (113) has a sixth inner diameter (1136), and the sixth inner diameter (1136) is substantially equal to the fifth inner diameter (1125) so that a junction where the front end pipe body (112) and the rear end pipe body (113) connect with each other is coplanar.

15. The female connecter (7) according to Claim 14, wherein the hermetic ring (12) is disposed at a location of the inner wall of the front end pipe body (112) that is between the third inner diameter (1123) and the fourth inner diameter (1124).
